(19) Europäisches Patentamt
European Patent Office
Office européen des brevets

(11) Veröffentlichungsnummer: **0 299 211 B1**

(12) **EUROPÄISCHE PATENTSCHRIFT**

(45) Veröffentlichungstag der Patentschrift: **22.04.92**

(51) Int. Cl.[5]: **A61K 9/24**, A61K 31/44

(21) Anmeldenummer: **88109420.5**

(22) Anmeldetag: **14.06.88**

(54) **DHP-Manteltablette.**

(30) Priorität: **24.06.87 DE 3720757**

(43) Veröffentlichungstag der Anmeldung:
**18.01.89 Patentblatt 89/03**

(45) Bekanntmachung des Hinweises auf die Patenterteilung:
**22.04.92 Patentblatt 92/17**

(84) Benannte Vertragsstaaten:
**AT BE CH DE ES FR GB GR IT LI NL SE**

(56) Entgegenhaltungen:
**FR-A- 2 331 375**
**GB-A- 1 144 915**

**CHEMICAL ABSTRACTS, Band 104, Nr. 20, 19 Mai 1986, Columbus, OH (US); Seite 391, Nr. 174662y**

**PHARMAZEUTISCHE INDUSTRIE, Band 24, Nr. 9, September 1962; T.WAGNER, Seiten 417-422**

(73) Patentinhaber: **BAYER AG**
**W-5090 Leverkusen 1 Bayerwerk(DE)**

(72) Erfinder: **Ohm, Andreas, Dr.**
**Am Röttgen 42**
**W-4040 Neuss 1(DE)**
Erfinder: **Luchtenberg, Helmut, Dr.**
**Windmühlenstr. 11a**
**W-5216 Niederkassel(DE)**
Erfinder: **Maegata, Shinji**
**1072-15, Oharanaka Koka-cho**
**Koka-gun Shiga Prefecture 520-34(JP)**
Erfinder: **Opitz, Wolfgang, Dr.**
**Holzbachtalstr. 60**
**W-5063 Overath(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Die Erfindung betrifft feste Arzneizubereitungen mit lang anhaltender Wirkung für Dihydropyridine in Form einer Manteltablette sowie Verfahren zu ihrer Herstellung.

Wirkstoffe aus der Stoffklasse der Dihydropyridine und ihre Verwendung als Herz- und Kreislaufmittel sind bereits bekannt (vgl. Brit. Pat. 1 173 862, Brit. Pat. 1 358 951, US-Pat. 4 256 749, DE-OS 3 311 003 und US-Pat. 4 264 611). Bei der galenischen Zubereitung dieser potenten Wirkstoffe treten häufig Schwierigkeiten auf, da die Stoffe nur eine sehr geringe Löslichkeit besitzen, häufig lichtempfindlich sind und ihre Resorbierbarkeit in biologischen Systemen häufig zu Problemen führt. Es wurden zahlreiche Versuche unternommen, optimale galenische Zubereitungen herzustellen, die die Bioverfügbarkeit dieser potenten Wirkstoffe verbessert. So wurden z.B. einige Wirkstoffe in speziellen organischen Lösungsmittelsystemen gelöst und in Gelatinekapseln eingefüllt, um einen schnellen und effektiven Wirkungseintritt zu gewährleisten (vgl. Brit. Pat. 1 362 627). Es wurde auch versucht, Dihydropyridine wie Nifedipin unter Verwendung von wasserlöslichen Polymeren in Copräzipitate bzw. "feste Lösungen" zu überführen, um die Bioverfügbarkeit zu verbessern (vgl. Brit. Pat. 1 579 818).

Für die Behandlung von Krankheiten, die über längere Zeiträume behandelt werden müssen, wie z.B. Hypertonie ist es wünschenswert, die Häufigkeit der Einnahme von Medikamenten so gering wie möglich zu halten. Dies ist nicht nur angenehmer für den Patienten, sondern es erhöht auch die Behandlungssicherheit, indem es die Nachteile unregelmäßiger Einnahmen vermindert und zu einem gleichmäßigen Wirkstoffkonzentrations-Zeitprofil im Körper führt. Dadurch wird gleichzeitig das Risiko von unerwünschten Über- bzw. Unterdosierungen minimiert.

Sowohl für den Arzt als auch für den Patienten besteht ein Bedürfnis, z.B. für die Dauertherapie von Kreislauferkrankungen, die hochwirksamen Dihydropyridine in einer Form zur Verfügung gestellt zu bekommen, daß eine einmal tägliche Applikation zur Krankheitsbehandlung ausreicht. Für Dihydropyridine wurden bereits Arzneizubereitungen mit verzögerter Wirkstoff-Freigabe (Retard-Formen) beschrieben. So wurde z.B. versucht, durch eine spezielle Korngrößenverteilung des kristallinen Wirkstoffs bzw. durch eine ausgewählte spezifische Oberfläche der Wirkstoffkristalle eine langsam freisetzende Zubereitung herzustellen (vgl. DE-OS 3 033 919). Weiterhin wurden spezielle Tablettenzubereitungen vorgeschlagen, die nach dem Prinzip der osmotischen Pumpe den Wirkstoff aus dem Inneren einer Tablette, die mit einer semipermeablen Lackschicht umgeben ist, durch eine vorgegebene Öffnung über einen längeren Zeitraum freisetzen und somit einen Retard-Effekt erzielen (vgl. US-PS 3 916 899).

Die bisher bekannten Zubereitungsformen mit verzögerter Wirkstoffabgabe, insbesondere solche für Dihydropyridine, weisen eine Reihe von Nachteilen auf. Ihre Retard-Wirkung ist z. B. bei einigen Formen nur auf einige Stunden beschränkt, so daß der Patient in der Regel nach wie vor zwei- oder mehrmals täglich applizieren muß. Nach einigen Stunden läßt die Freisetzungsgeschwindigkeit des Wirkstoffs deutlich nach, so daß auch die Blutspiegel unter die erforderliche Effektivitätsgrenze absinken können.

In der DE-OS 26 51 176 bzw. in FR-A 2331375 werden Pellets mit kontrollierter Wirkstoffabgabe beschrieben. Die dort genannten Formulierungen unterscheiden sich von den erfindungsgemäßen Manteltabletten grundsätzlich grundsätzlich schon dadurch, daß die Formlinge zwingend einen Konzentrationsgradienten in der Hülle besitzen und nur in aufwendigen Verfahren durch kontinuierliches Aufbringen vieler Schichten erhalten werden können, während die erfindungsgemäße Tablette durch einfaches Verpressen hergestellt wird. Ein weiterer wesentlicher Unterschied liegt darin, daß die kugelförmigen Pellets gemäß dieser Offenlegungsschrift, selbst wenn sie in Tablettendimensionen hergestellt werden, eine terminalabnehmende Freisetzungsrate zeigen im Gegensatz zu der terminal sprunghaft ansteigenden Freisetzungsrate der erfindungsgemäßen Manteltabletten. In den dort genannten Ausführungs beispielen werden nur leicht lösliche Wirkstoffe verwandt und alle Beispiele beschreiben die Herstellung der Pelletschichten mit lipophilen Retardierungsmitteln. Die erfindungsgemäße Verwendung von hydrophilen Polymeren, insbesondere von Hydroxypropylcellulose ist nach den Ausführungsbeispielen dieser Offenlegungsschrift praktisch nicht durchzuführen.

Bei dem oben erwähnten osmotischen System kann es im Magen oder Darmtrakt je nach eingesetzter Kapselfüllung zu lokalen Irritationen des Gewebes durch überhöhte Konzentration kommen. Weiterhin ist auch bei diesem osmotischen Retardierungsprinzip, das über einen relativ langen Zeitraum einen linearen Freisetzungsverlauf gewährleisten soll, eine Abflachung der Freisetzungskurve im terminalen Bereich zu beobachten. Bedingt durch die Natur des osmotischen Systems verbleibt ein Teil des Wirkstoffs in der Arzneiform und steht somit nicht für die gewünschte Resorption zur Verfügung. Eine weiterer Nachteil dieses Systems ist das verzögerte Einsetzen der Wirkstoffabgabe nach der Applikation, die teilweise erst nach ca. 2 Stunden beginnt. Die Herstellung dieser Arzneiform ist zudem sehr aufwendig, da hierbei organische Lösungsmittel im Herstellungsprozeß eingesetzt werden müssen und die Lackschicht jeder

Tablette einzeln mit Hilfe eines Laserstrahls durchbohrt werden muß.

Es wurde nun gefunden, daß feste Arzneizubereitungen mit langanhaltender Wirkung in Form einer Manteltablette, die einen schwer löslichen Dihydropyridin-Wirkstoff der allgemeinen Formel I

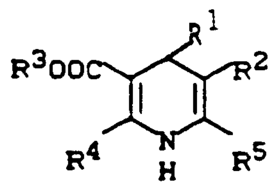 I,

enthalten, in welcher

$R^1$ für einen Phenylrest steht, der durch ein oder zwei gleiche oder verschiedene Substituenten aus der Gruppe Nitro, Halogen oder Trifluormethyl substituiert ist, oder für einen Rest aus der Gruppe

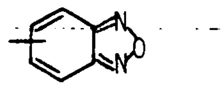

oder

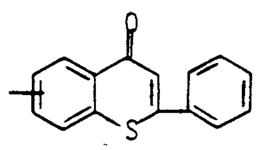

steht,

$R^2$ für eine Nitrogruppe steht oder für den Rest $COOR_6$ steht, wobei

$R_6$ Alkyl mit 1 bis 10 C-Atomen bedeutet, welches gegebenenfalls substituiert ist durch Alkoxy mit 1 bis 4 C-Atomen oder durch ein oder mehrere Halogene

oder wobei

$R^2$ gemeinsam mit $R^5$ für die Lactongruppe $-CO-O-CH_2$-steht,

$R^3$ für Alkyl mit 1 bis 10 C-Atomen steht, welches gegebenenfalls substituiert ist durch Alkoxy mit 1 bis 4 C-Atomen oder durch ein oder mehrere Fluor-Atome und

$R^4$ und $R^5$ gleich oder verschieden sind und jeweils für Alkyl mit 1 bis 4 C-Atomen, welches gegebenenfalls durch Hydroxy substituiert ist, stehen,

wobei die Manteltablette

a) aus einem Kern besteht, der 10 bis 40 % des Dihydropyridin-Wirkstoffs in schnell freisetzender Form enthält und

b) aus einem um den Kern liegenden Mantel besteht, der 60 bis 90 % des Dihydropyridin-Wirkstoffs in langsam freisetzender Form enthält, eine überraschend lang anhaltende Effektivität zeigen.

Als besonders bevorzugte Wirkstoffe seien genannt, Nifedipin, Nitrendipin, Nimodipin und Nisoldipin.

Je nach Art des Wirkstoffs enthalten die erfindungsgemäßen Manteltabletten insgesamt Vorzugsweise 1 bis 200 mg, insbesondere 10 bis 150 mg mindestens eines Wirkstoffs aus der Klasse der Dihydropyridine.

Der schnell freisetzende Kern der Manteltablette enthält den Wirkstoff vorzugsweise in amorpher Form oder in fein gemahlener bzw. mikronisierter kristalliner Form. Bei Verwendung Von kristallinem Wirkstoff wird die Freisetzungsrate Vorzugsweise durch die Zugabe von gut wasserlöslichen Hilfsstoffen und durch Änderung der Korngrößenverteilung des Wirkstoffs beeinflußt.

Als Tablettenkerne mit schneller Freisetzung werden vorzugsweise solche Kerne verstanden, die den Dihydropyridinwirkstoff in einer Stunde, vorzugsweise in 30 Minuten, zu 75 % freisetzen.

Falls der schnell freisetzende Kern amorphes Dihydropyridin enthält, wird dieses vorzugsweise gemeinsam mit wasserlöslichen Polymeren wie Polyvinylpoyrrolidon, Methylcellulose, Hydroxypropylcellulose oder Hydroxypropylmethylcellulose in organischem Lösungsmittel gelöst. Hierbei ist es zweckmäßig, auf 1 Gew.-Teil Dihydropyridin 2 bis 10 Gew.-Teile, insbesondere 3 bis 8 Gew.-Teile der wasserlöslichen Polymere einzusetzen und hieraus entsprechende Copräzipitate herzustellen.

Falls der schnell freisetzende Kern Dihydropyridine in kristalliner Form enthält, werden vorzugsweise

Dihydropyridinkristalle mit einer maximalen mittleren Korngröße von ca. 25 µm insbesondere einer maximalen mittleren Korngröße von ca. 15 µm eingesetzt. Die Bestimmung der Korngröße erfolgt nach der Cilas-Methode (Lit.: A. Buerkholz et al, Part. Charact. 1, 1984, 153-160, "Laser defraction spectrometers/experience in particle size analysis".).

Bei Verwendung von kristallinem Dihydropyridin im Kern ist die Zugabe von leicht wasserlöslichen Hilfsstoffen wie z.B. Lactose zweckmäßig. Ebenfalls kann durch den Einsatz von Sprengmitteln, wie z.B. quervernetztem Polyvinylpyrrolidon (PVP), oder durch oberflächenaktive Substanzen, wie z.B. Natriumlaurylsulfat, die Freisetzungsrate beschleunigt werden.

Die Herstellung dieses schnell freisetzenden Kerns erfolgt nach üblichen Methoden (vgl. DE-OS 3 415 128 und DE-OS 3 142 853 oder Brit. Pat. 1 579 818).

Der Mantel der Tablette enthält 10 bis 99 %, vorzugsweise 20 bis 90 % des Gesamtmantelgewichts an hydrophilen gelbildenden Polymeren.

Als hydrophile gelbildende Polymere sind zum Beispiel modifizierte Stärke oder celluloseartige Substanzen wie z.B. Methylcellulose, Hydroxypropylmethylcellulose, Hydroxypropylcellulose und Natriumcarboxymethylcellulose geeignet. Als besonders bevorzugt sei Hydroxypropylcellulose (HPC) genannt (vgl.: Hagers Handbuch der pharmazeutischen Praxis, Band 7, Teil B, (1977) 130-141).

Erfindungsgemäß lassen sich verschiedene Arten von HPC verwenden, die sich jeweils in ihrer Viskosität unterscheiden, z.B. HPC-L (niedere Viskosität von ca. 6-10 mPa.s), HPC-M (mittlere Viskosität von ca. 150 mPa.s) und HPC-H (hohe Viskosität von ca. 1000-4000 mPa.s). Die Freisetzungsrate kann gesteuert werden über die unterschiedlichen Viskositätsgrade, wobei die Freisetzungsrate sich erhöht, wenn niederviskose Qualitäten eingesetzt werden und langsamer wird beim Einsatz hochviskoser Typen.

Gegebenenfalls ist es zweckmäßig, einen Teil des Wirkstoffs in Form einer äußeren Schicht der Manteltablette als Initialdosis mit den bekannten Techniken und Hilfsstoffen aufzubringen.

Übliche bekannte galenische Maßnahmen wie z.B. das Lackieren des Kerns mit einer magensaftresistenten Schicht, die Verwendung von Geschmacks- und Aromastoffen und Schmiermitteln und üblichen Hilfsmitteln, die dem galenischen Fachmann geläufig sind, können selbstverständlich auch bei der erfindungsgemäßen Manteltablette eingesetzt und verwendet werden.

Es sei ausdrücklich darauf hingewiesen, daß die erfindungsgemäße Manteltablette sich von den bisher bekannten Manteltabletten dadurch unterscheidet, daß der Mantel den Wirkstoff in langsam freisetzender Form enthält und der Kern den Wirkstoff in schnell freisetzender Form enthält.

Aus dem Stand der Technik sind bereits Mehrschicht-Tabletten auf Basis von Casein-Matrices beschrieben, die zwei oder drei Schichten enthalten, die jeweils ihrerseits Wirkstoffe enthalten können (vgl. US-Pat. 3 184 386). Die dort beschriebenen Tabletten enthalten im äußeren Mantel eine schnell freisetzende Zubereitung wobei der Kern primär die Funktion hat, die für die Freisetzung relevante Oberfläche der äußeren wirkstoffenthaltenden Schicht nicht zu klein werden zu lassen. Diese Patentschrift enthält jedoch keinen Hinweis darauf, daß der Kern der Zubereitung den Wirkstoff in schnell freisetzender Form enthält. Vielmehr sind sowohl der mittlere Mantel als auch der Kern in den Beispielen als langsam freisetzende Formen beschrieben.

Auch in der US-Pat. 3 558 768 werden Manteltabletten beschrieben, die sowohl im Kern als auch im Mantel Wirkstoffe in langsam freisetzender Form enthalten. Die Freisetzungsgeschwindigkeiten können gemäß dieser US-Patentschrift verschieden sein, wobei es sich aber auf jeden Fall um langsam freisetzende Formen handelt.

In Chem. Abs. 104 (1986) Nr. 174662y werden Nifedipin-Zubereitungen beschrieben, die zunächst eine schnelle Wirkstoffabgabe (Initialdosis) gewährleisten, welche dann mit einer verzögert freisetzenden Wirkstoffabgabe (Erhaltungsdosis) kombiniert wird. Durch solche Formulierungen kann eine schnelle Wirkstoffabgabe zur Erreichung eines wirksamen Plasmaspiegels erreicht werden, der dann durch die verzögerte Abgabe der zweiten Wirkstoffdosis aufrecht erhalten wird. Dieses Prinzip sichert lediglich das schnelle Erreichen wirksamer Spiegel direkt nach Einnahme. Es ist hiernach jedoch nicht möglich, bei einmaliger Verabreichung zu einem gewünschten späteren Zeitpunkt höhere Wirkspiegel zu erreichen. Nur durch die erfindungsgemäße Formulierung kann sichergestellt werden, daß auch noch nach einem längeren Zeitraum nach der Applikation zu einem bestimmten Zeitpunkt ein erhöhter Wirkstoffspiegel zur Verfügung gestellt wird.

Durch das Prinzip der erfindungsgemäßen Manteltablette werden die bisher üblichen Nachteile von normalen Retard-Tabletten und auch von bisher bekannten Mehrschicht- oder Manteltabletten bzw. von Zubereitungsformen die auf dem osmotischen Prinzip beruhen, vermieden. Insbesondere wird vermieden, daß die Freisetzungsrate des Wirkstoffs gegen Ende der Auflösung der Tabletten immer kleiner wird und damit die Plasmaspiegel absinken.

Die durch Verringerung des Volumens der Tablette abnehmende Freisetzungsrate normaler Retard-Tabletten wird durch die schnell freisetzende Wirkung des Kerns der erfindungsgemäßen Manteltablette mehr als kompensiert. Gleichzeitig wird eine vollständige Freisetzung des Wirkstoffs erreicht im Vergleich zu osmotischen Systemen.

Durch die beschleunigte Freisetzungsrate im terminalen Bereich bzw. den längeren linearen Freisetzungsverlauf unterscheidet sich die erfindungsgemäße Formulierung von allen bisher bekannten Retardierungsprinzipien für feste Arzneiformen. Eine eventuelle Minderresorption des applizierten Arzneistoffes in tieferen Darmabschnitten, z.B. bedingt durch Behinderung der Diffusion, kann dadurch besser ausgeglichen werden. Als weitere Vorteile sind das rasche Anfluten des Wirkstoffs nach Applikation unter Vermeidung einer Verzögerungsphase sowie die einfache Herstellungstechnologie zu nennen.

Als weiterer Vorteil der erfindungsgemäßen Manteltablette sei genannt, daß sie insbesondere für solche Wirkstoffe, die im unteren Gastrointestinalbereich, z. B. im Dickdarm, eine hohe Resorptionsrate zeigen, besonders nützlich ist. Die erfindungsgemäße Zubereitung kann für solche Wirkstoffe eine erhöhte Bioverfügbarkeit gewährleisten.

Die Herstellung der erfindungsgemäßen Zubereitungen erfolgt nach üblichen Methoden, beispielsweise durch folgendes Verfahren:

A) Kernherstellung

Der Wirkstoff und die anderen Hilfsstoffe für den Kern werden gemischt und nach üblichen Methoden granuliert durch Zugabe einer wäßrigen Binderlösung, z. B. in einem Planetenmischer, einem Hochgeschwindigkeitsmischer oder einem Wirbelschichtgranulator. Das erhaltene Granulat wird getrocknet, vorzugsweise in einem Wirbelschichttrockner, anschließend gesiebt und mit Magnesiumstearat vermischt und anschließend zu Tabletten verpreßt. Alternativ kann die Herstellung auch durch direktes Verpressen der Bestandteile erfolgen, wobei gegebenenfalls der erhaltene Kern nach üblichen Methoden, z. B. in einem Lackierkessel mit einer Lackschicht versehen werden kann.

B) Herstellung des Mantelgranulates

Das Granulat wird nach üblichen Methoden hergestellt, vorzugsweise in einem Wirbelschichtgranulator durch Einsprühen einer wäßrigen Suspension, welche den Wirkstoff und ein Bindemittel enthält, auf die festen Bestandteile, welche anschließend getrocknet, gesiebt und mit einem Gleitmittel wie z. B. Magnesiumstearat vermischt werden.

C) Tablettierung der Manteltablette

Das Aufpressen des Mantels auf den Kern erfolgt auf handelsüblichen Manteltablettenpressen (z. B. Presscoater der Firma Kilian oder Manesty). Falls erforderlich, kann die Tablette anschließend noch mit einem Lack überzogen werden, wobei gegebenenfalls Lichtschutzmittel, Geschmacksverbesserer oder eine Initialdosis des Wirkstoffs in diese Lackschicht eingebracht werden kann, wobei die Menge des so eingebrachten Wirkstoffs maximal 20 % der gesamten Menge der gebrauchsfertigen Formulierung beträgt.

Im Hinblick auf das seit langem bestehende Bedürfnis nach Arzneizubereitungsformen mit lang anhaltender Wirkung ist es mehr als überraschend, daß bisher niemand die einfach herstellbare und sehr effektvolle erfindungsgemäße Manteltablette mit schnell freisetzendem Kern beschrieben oder hergestellt hat. Durch die vorliegende Erfindung wird der Patient in die Lage versetzt, das Medikament nur einmal täglich applizieren zu müssen, was insbesondere bei Dauertherapie eine sicherere und angenehmere Behandlungsart darstellt.

Die Kurven der Abbildung 1 zeigen für einige erfindungsgemäße Beispiele das Prinzip des über einige Stunden langsam freisetzenden Mantels und des anschließend schnell freisetzenden Kerns.

Ausführungsbeispiele

Beispiel 1

A) Kern

50 g kristallines Nifedipin (mittlere Korngröße 5 µm) werden mit 388 g Milchzucker und 150 g Maisstärke gemischt, in einem Kleister aus 10 g Stärke und 140 g heißem Wasser granuliert und anschließend getrocknet. Das Granulat wird gesiebt und mit 50 g mikrokristalliner Cellulose und 2 g

Magnesiumstearat gemischt. Diese Mischung wird zu 65 mg schweren Tabletten mit einem Durchmesser von 6 mm verpreßt. Die Kerne werden mit einer organischen Lösung aus Hydroxypropylmethylcellulosephthalat magensaftresistent lackiert. Die lackierte Tablette wiegt 72 mg

B) Mantel

250 g Nifedipin werden mit 400 g Milchzucker, 16 g kolloidalem Siliciumdioxid, 700 g Hydroxypropylcellulose Typ M, 1747 g Hydroxypropylcellulose Typ L und 320 g Zitronensäure gemischt und in einem Wirbelschichtgranulator mit einer Lösung aus 20 g Hydroxypropylcellulose Typ L granuliert. Das getrocknete und gesiebte Granulat wird mit 27 g Magnesiumstearat gemischt.

Dieses Mantelgranulat und die unter A) beschriebenen Kerne werden auf einer Mantel-Tablettenpresse zu 420 mg schweren Manteltabletten verpreßt mit einem Durchmesser von 10 mm. Die Tabletten werden anschließend mit einer Lackdispersion aus Hydroxypropylmethylcellulose, Polyethylenglykol, Titandioxid und Eisenoxid rot lackiert.

Beispiel 2

A) Kern

Herstellung wie in Beispiel 1

B) Mantel

400 g Milchzucker werden mit 17 g kolloidalem Siliciumdioxid, 2196 g Hydroxypropylcellulose Typ L, 250 g Hydroxypropylcellulose Typ M und 320 g Citronensäure gemischt und in einem Wirbelschichtgranulator mit einer wäßrigen Suspension aus 250 g Nifedipin und 20 g Hydroxypropylcellulose Typ L granuliert. Das getrocknete Granulat wird gesiebt und mit 27 g Magnesiumstearat gemischt.

Dieses Mantelgranulat und die unter A) beschriebenen Kerne werden auf einer Mantel-Tablettenpresse zu 420 mg schweren Mantel-Tabletten verpreßt mit einem Durchmesser von 10 mm. Die Tabletten werden anschließend mit einer Lackdispersion aus Hydroxy propylmethylcellulose, Polyethylenglykol, Titandioxid und Eisenoxid rot lackiert.

Beispiel 3

A) Kern

50 g kristallines Nifedipin (mittlere Korngröße 8 µm) werden mit 291 g Milchzucker, 162,5 g Maisstärke gemischt und mit einem Kleister aus 7,5 g Maisstärke in 100 g heißem Wasser granuliert. Das Granulat wird getrocknet, gesiebt und anschließend mit 1,5 g Magnesiumstearat und 37,5 g mikrokristalliner Cellulose gemischt. Diese Mischung wird zu 55 mg schweren Kernen mit einem Durchmesser von 5,5 mm verpreßt.

B) Mantel

400 g Milchzucker werden mit 17 g kolloidalem Siliciumdioxid, 1105 g HPC Typ L, 443 g HPC Typ M und 202 g Citronensäure gemischt und mit einer wäßrigen Suspension bestehend aus 250 g Nifedipin und 16 g HPC Typ L granuliert. Das Granulat wird getrocknet und gesiebt und mit 17 g Magnesiumstearat gemischt.

Aus diesem Mantelgranulat und den Kernen werden Mantel-Tabletten mit einem Gewicht von 300 mg und einem Durchmesser von 9 mm hergestellt. Die Tabletten werden anschließend lackiert gemäß Beispiel 1.

Beispiel 4

A) Kern

Herstellung wie in Beispiel 3.

Die Kerne werden mit einer organischen Lösung aus Hydroxypropylmethylcellulosephthalat magensaftresistent lackiert. Die lackierten Tabletten wiegen 60 mg.

B) Mantel

250 g Nifedipin werden mit 400 g Milchzucker, 17 g kolloidalem Siliciumdioxid, 1155 g HPC Typ L, 343 g HPC Typ M und 202 g Citronensäure gemischt und mit einer wäßrigen Lösung aus 16 g HPC Typ L granuliert. Das Granulat wird getrocknet, gesiebt und mit 17 g Magnesiumstearat gemischt.

Aus diesem Mantelgranulat und den Kernen werden Mantel-Tabletten mit einem Gewicht von 300 mg und einem Durchmesser von 9 mm hergestellt. Die Tabletten werden anschließend lackiert gemäß Beispiel 1.

Beispiel 5

A) Kern

250 g quervernetztes Polyvinylpyrrolidon und 197 g mikrokristalline Cellulose werden gemischt und mit einer Lösung aus 30 g Nifedipin und 150 g Polyvinylpyrrolidon 25 in 350 g Aceton granuliert. Das Granulat wird getrocknet und gesiebt und mit 3 g Magnesiumstearat verpreßt. Diese Mischung wird zu 65 mg schweren Tabletten mit einem Durchmesser von 6 mm verpreßt. Die Kerne werden mit einer organischen Lösung aus Hydroxypropylmethylcellulosephthalat lackiert. Die lackierten Tabletten wiegen 72 mg.

B) Mantel

Das Mantelgranulat wird analog Beispiel 1 hergestellt.

Die weitere Verarbeitung erfolgt gemäß Beispiel 1.

Beispiel 6

A) Kern

Herstellung wie in Beispiel 3.

B) Mantel

200 g Nifedipin werden mit 350 g Milchzucker, 17 g kolloidalem Siliciumdioxid, 1105 g HPC Typ L, 443 g HPC Typ M und 202 g Citronensäure gemischt und mit einer wäßrigen Lösung aus 16 g HPC Typ L granuliert. Das Granulat wird getrocknet und gesiebt und mit 12 g Magnesiumstearat gemischt.

Aus diesem Mantelgranulat und den Kernen werden Mantel-Tabletten mit einem Gewicht von 290 mg gepreßt.

Auf diese Tabletten werden aus einer wäßrigen Dispersion mit Hydroxypropylmethylcellulose und Polyethylenglykol 5 mg Nifedipin pro Tablette auflackiert. Diese Tabletten werden anschließend mit einem Lichtschutzlack analog Beispiel 1 überzogen.

Beispiel 7

A) Kern

50 g kristallines Nifedipin (mittlere Korngröße 10 µm) werden mit 600 g Milchzucker und 228 g Maisstärke gemischt und mit einem Kleister aus 20 g Stärke und 320 g Wasser granuliert und anschließend getrocknet. Das Granulat wird gesiebt und mit 2 g Magnesiumstearat gemischt und zu 90 mg schweren Tabletten mit einem Durchmesser von 7 mm verpreßt. Die Kerne werden mit einer organischen Lösung aus Hydroxypropylmethylcellulosephthalat lackiert. Die lackierten Tabletten wiegen 97 mg.

B) Mantel

250 g Nifedipin werden mit 400 g Milchzucker, 16 g kolloidalem Siliciumdioxid gemischt und mit einer Lösung aus 16 g Hydroxypropylcellulose Typ L in Wasser granuliert. Das getrocknete und gesiebte Granulat wird mit 900 g Hydroxypropylcellulose Typ M, 2387 g Hydroxypropylcellulose Typ L, 400 g Zitronensäure und 61 g Magnesiumstearat gemischt.

Dieses Mantelgranulat und die unter A) beschriebenen Kerne werden auf einer Mantel-Tablettenpresse

zu 540 mg schweren Tabletten mit einem Durchmesser von 11 mm verpreßt. Die Tabletten werden anschließend mit einer Lackdispersion aus Hydroxypropylmethylcellulose, Polyethylenglykol, Titandioxid und Eisenoxid rot lackiert.

Beispiel 8

A) Kern

100 g kristallines Nifedipin (mittlere Korngröße 4 µm) werden mit 241 g Milchzucker und 162,5 g Maisstärke gemischt und mit einem Kleister aus 7,5 g Maisstärke in 100 g Wasser granuliert. Das getrocknete Granulat wird gesiebt, mit 1,5 g Magnesiumstearat und 37,5 g Avicel gemischt und zu 55 mg schweren Tabletten mit einem Durchmesser von 5,5 mm verpreßt.

B) Mantel

500 g Nifedipin werden mit 335 g Milchzucker, 16 g kolloidalem Siliciumdioxid gemischt und mit einer Lösung aus 33 g Hydroxypropylcellulose Typ L in Wasser granuliert. Das getrocknete Granulat wird gesiebt und mit 443 g Hydroxypropylcellulose Typ M, 1105 g Hydroxypropylcellulose Typ L und 18 g Magnesiumstearat gemischt.

Dieses Mantelgranulat und die unter A) beschriebenen Kerne werden auf einer Mantel-Tablettenpresse zu 300 mg schweren Tabletten mit einem Durchmesser von 9 mm verpreßt. Die Tabletten werden anschließend mit einer Lackdispersion aus Hydroxypropylmethylcellulose, Polyethylenglykol, Titandioxid und Eisenoxid rot lackiert.

In analoger Weise wurden die Beispiele 9 und 10 hergestellt.

Beispiel 9

| | | |
|---|---|---|
| Kern: | Nitrendipin mittl. Korngr. 6 µm | 5,0 mg |
| | Maisstärke feucht | 27,8 mg |
| | mikrokrist. Cellulose | 20,0 mg |
| | Lactose fein | 21,49 mg |
| | wird gemischt und anschließend granuliert mit: | |
| | Polyvinylpyrrolidon 25 | 5,0 mg |
| | Natriumlaurylsulfat | 0,5 mg |
| | FD + C Blue Lake No. 2 | 0,01 mg |
| | in wäßriger Suspension mittels üblicher Granulationsverfahren | |
| | nach Trocknung wird zugemischt | |
| | Magnesiumstearat | 0,2 mg |

**auf einer Tablettenpresse wird zu Kernen verpreßt:**

**Gewicht: 80 mg, Format: ø6 mm**

| | | |
|---|---|---|
| Mantel: | **Hydroxypropylcellulose Typ L** | **212,0 mg** |
| | **Hydroxypropylcellulose Typ M** | **82,0 mg** |
| | **Zitronensäure** | **146,0 mg** |

werden gemischt und granuliert mit einer wäßrigen Nitrendipin-haltigen Suspension entsprechend 25.0 mg Nitrendipin mittl. Korngröße 5 µm/Tablettenmantel (gegebenenfalls kann ein Teil der Hydroxypropylcellulose entnommen werden zur Einarbeitung in die Granulationsflüssigkeit)

| | |
|---|---|
| **Zumischen von Magnesiumstearat** | 5,0 mg |

Mit Hilfe einer Mantel-Tablettenpresse werden Manteltabletten hergestellt:
Gesamtgewicht: 550mg
Format: ∅10 mm

Beispiel 10

| | | |
|---|---|---|
| Kern: | Nitrendipin mittl. Korng. 5 µm | 5,0 mg |
| | mikrokrist. Cellulose | 17,5 mg |
| | Lactose fein | 6,4 mg |
| | Maisstärke | 7,5 mg |

mischen und granulieren mit

| | |
|---|---|
| Polyvinylpyrrolidon 25 | 3,0 mg |
| Natriumlaurylsulfat | 0,5 mg |

in wäßriger Lösung mittels üblicher Granulationsverfahren

nach Trocknung wird zugemischt

| | |
|---|---|
| Magnesiumstearat | 0,1 mg |

auf einer Tablettenpresse wird zu Kernen verpreßt:

Gewicht: 40 mg, Format: ø5,5 mm

| | | |
|---|---|---|
| Mantel: | Nitrendipin mikrofein | 25,0 mg |
| | Hydroxypropylcellulose Typ L | 221,0 mg |

werden gemischt und granuliert (gegebenenfalls kann ein Teil der Hydroxypropylcellulose entnommen werden zur Einarbeitung in die Granulationsflüssigkeit)

Zumischen von

| | |
|---|---|
| Magnesiumstearat | 7,0 mg |

Mit Hilfe einer Mantel-Tablettenpresse werden Manteltabletten hergestellt:

Gesamtgewicht: 293 mg
Format: ø9 mm

**Beispiel 11**

| | | |
|---|---|---|
| Kern: | Nitrendipin mittl. Korngr. 10 µm | 2,5 mg |
| | Maisstärke feucht | 23,0 mg |
| | mikrokrist. Cellulose | 20,0 mg |
| | Lactose fein | 21,5 mg |
| | Plasdone XL | 7,3 mg |

wird granuliert mit einer wäßrigen Lösung von:

| | |
|---|---|
| Polyvinylpyrrolidon 25 | 5 mg |
| Natriumlaurylsulfat | 0,5 mg |

nach Trocknung wird zugemischt

| | | |
|---|---|---|
| | Magnesiumstearat | 0,2 mg |

auf einer Tablettenpresse wird zu Kernen verpreßt.

Gewicht: 80 mg, Format: ø6 mm

| | | |
|---|---|---|
| Mantel: | Nisoldipin mikrofein | 12,5 mg |
| | Hydroxypropylcellulose Typ L | 212 mg |
| | Hydroxypropylcellulose Typ M | 82 mg |
| | Lactose fein | 158,5 mg |

werden gemischt und granuliert mit Wasser

| | | |
|---|---|---|
| | Zumischen von Magnesiumstearat | 12 mg |

Mit Hilfe einer Mantel-Tablettenpresse werden Manteltabletten hergestellt:
Gesamtgewicht: 557 mg
Format: ∅10 mm

Beispiel 12

A) Kern

Die Herstellung erfolgt gemäß Beispiel 8 wobei anstelle von 100 g Nifedipin und 241 g Milchzucker jetzt 200 g Nimodipin und 141 g Milchzucker eingesetzt werden.

B) Mantel

Herstellung analog Beispiel 8 wobei anstelle von 500 g Nifedipin und 335 g Milchzucker jetzt 600 g Nimodipin und 235 g Milchzucker eingesetzt werden.

Die Lackierung erfolgt ebenfalls analog Beispiel 8 jedoch ohne die Verwendung von Eisenoxid rot.

Beispiel 13

A) Kern

Die Herstellung erfolgt analog Beispiel 8 wobei anstelle von 100 g Nifedipin und 241 g Lactose jetzt 50 g Nifedipin, 150 g Nisoldipin und 141 g Lactose eingesetzt werden.

B) Mantel

Die Herstellung erfolgt analog Beispiel 8 wobei anstelle von 500 g Nifedipin jetzt 200 g Nifedipin und 300 g Nisoldipin eingesetzt werden. Gleichzeitig wird anstelle von 443 g HPC-M und 1105 g HPC-L jetzt 518 g HPC-M und 1030 g HPC-L eingesetzt.

Beispiel 14

| | | |
|---|---|---|
| Kern: | Nitrendipin mittl. Korng. 5 µm | 8,0 mg |
| | mikrokrist. Cellulose | 12,0 mg |
| | Lactose fein | 4,0 mg |
| | Plasdone XL | 15,0 mg |

mischen und granulieren mit

| | |
|---|---|
| Polyvinylpyrrolidon 25 | 2,0 mg |
| Natriumlaurylsulfat | 0,8 mg |

in wäßriger Lösung mittels üblicher Granulationsverfahren

nach Trocknung wird zugemischt

| | |
|---|---|
| Magnesiumstearat | 0,2 mg |

auf einer Tablettenpresse wird zu Kernen verpreßt: Gewicht: 42 mg, Format: Ø 5 mm

Mantel:

| | | |
|---|---|---|
| Nitrendipin mikrofein | | 32,0 mg |
| Hydroxypropylcellulose | Typ L | 77,0 mg |
| | Typ M | 77,0 mg |
| Lactose fein | | 92,5 mg |

werden gemischt und granuliert (gegebenenfalls kann ein Teil der Hydroxypropylcellulose entnommen werden zur Einarbeitung in die Granulationsflüssigkeit)

Zumischen von Magnesiumstearat 1,5 mg

Mit Hilfe einer Mantel-Tablettenpresse werden Manteltabletten hergestellt: Gesamtgewicht: 322,0 mg; Format: Ø 9 mm

Beispiel 15

A) Kern

50 g Nifedipin (mittlerer Teilchendurchmesser 5 µm) werden gemischt mit 170 g Lactose und 173,5 g Maisstärke. Diese Mischung wird granuliert mit einer wäßrigen Paste von 5 g Maisstärke. Nach Trocknen und Sieben werden 1,5 g Magnesiumstearat, 50 g Plasdone XL und 50 g Avicel zugefügt und das Granulat zu Tabletten mit einem Durchmesser von 5 mm und einem Gewicht von 50 mg verpreßt.

B) Mantelgranulat

1.101 g Hydroxypropylcellulose Typ L, 755 g Hydroxypropylcellulose Typ M und 341 g Lactose werden mit 16 g kolloidem Kieselgel gemischt und anschließend mit einer wäßrigen Suspension von 250 g Nifedipin und 20 g Hydroxypropylcellulose Typ L granuliert. Das Granulat wird getrocknet, gesiebt und mit 17 g Magnesiumstearat gemischt zu Tabletten mit einem Gewicht von 300 mg verpreßt, die einen Tablettendurchmesser von 9 mm haben. Die Tabletten werden anschließend mit einer wäßrigen Suspension von Hydroxypropylmethylcellulose, Polyethylenglykol, Titaniumdioxid und rotem Eisenoxid (Lichtschutzmittel) lackiert.

Beispiel 16

A) Kern

100 g Nifedipin mit einem mittleren Teilchendurchmesser von 5 µm werden mit 160 g Lactose und 148,8 g Maisstärke gemischt und anschließend mit einer wäßrigen Paste von 5 g Maisstärke granuliert. Nach Trocknen und Sieben werden 1,3 g Magnesiumstearat, 50 g Plasdone XL und 34,9 g Avicel zugefügt und das erhaltene Granulat zu 50 mg schweren Tabletten mit einem Durchmesser von 5 mm verpreßt.

B) Mantelgranulat

1.010 g Hydroxypropylcellulose Typ L und 628 g Hydroxypropylcellulose Typ M werden mit 289 g Lactose und 16 g kolloidem Kieselgel gemischt und mit einer wäßrigen Suspension aus 500 g Nifedipin und 40 g HPC Typ L granuliert. Nach dem Trocknen und Sieben wird das Granulat mit 17 g Magnesiumstearat gemischt und zu Tabletten mit einem Gewicht von 300 mg und einem Durchmesser von 9 mm verpreßt. Die erhaltenen Tabletten werden anschließend mit einer wäßrigen Suspension aus Hydroxypropylmethylcellulose, Polyethylenglykol, Titaniumdioxid und rotem Eisenoxid (Lichtschutz) lackiert.

Beispiel 17

A) Kern

150 g Nifedipin (Teilchengröße 5 µm) werden mit 130 g Lactose und 124 g Maisstärke vermischt und mit einer wäßrigen Paste aus 5 g Maisstärke granuliert. Nach Trocknen und Sieben werden 1 g Magnesiumstearat, 50 g Plasdone XL und 40 g Avicel zugefügt und das Granulat zu 50 mg schweren Kernen mit einem Durchmesser von 5 mm verpreßt.

B) Mantelgranulat

780 g Hydroxypropylcellulose Typ L und 588 g Hydroxypropylcellulose Typ M werden mit 289 g Lactose und 16 g kolloider Kieselsäure gemischt und anschließend mit einer wäßrigen Suspension aus 750 g Nifedipin und 60 g Hydroxypropylcellulose Typ L granuliert. Nach Trocknen und Sieben wird das Granulat mit 17 g Magnesiumstearat gemischt und zu 300 mg schweren Tabletten mit einem Durchmesser von 9 mm verpreßt.

Beispiel 18

A) Kern

8 g Nitrendipin (mittlerer Teilchendurchmesser 5 µm) werden gemischt mit 4 g Lactose, 5 g quervernetztem PVPPund 12,3 g mikrokristalliner Cellulose und anschließend mit einer wäßrigen Lösung aus 1,8 g PVP und 0,8 g Natriumlaurylsulfat granuliert. Nach Trocknen und Sieben werden 0,1 g Magnesiumstearat zugegeben und die Mischung zu Tablettenkernen mit einem Gewicht von 42 mg und einem Durchmesser von 5 mm verpreßt.

B) Mantelgranulat

104,5 g Hydroxypropylcellulose Typ L und 40 g Hydroxypropylcellulose Typ M werden mit 88,5 g

Lactose gemischt und die Mischung mit einer wäßrigen Suspension aus 32 g Nitrendipin und 1,5 g Hydroxypropylcellulose L granuliert. Nach Trocknen und Sieben wird das Granulat mit 1,5 g Magnesiumstearat gemischt und zu 310 mg schweren Tabletten mit einem Durchmesser von 9 mm verpreßt. Anschließend werden die Tabletten mit einer wäßrigen Suspension aus Hydroxypropylmethylcellulose, Polyethylenglykol und Titaniumdioxid lackiert.

Beispiel 19

A) Kern

20 g Nitrendipin (mittlerer Teilchendurchmesser 5 µm) werden gemischt mit 15 g quervernetztem PVPP und 7,2 g mikrokristalliner Cellulose. Die Mischung wird mit einer wäßrigen Lösung von 1,8 g PVP und 0,9 g Natriumlaurylsulfat granuliert, anschließend getrocknet und gesiebt und mit 0,1 g Magnesiumstearat vermischt. Anschließend zu 45 mg schweren Tablettenkernen mit einem Durchmesser von 5 mm verpreßt.

B) Mantelgranulat

144,5 g Hydroxypropylcellulose Typ L und 97,5 g Lactose werden gemischt und mit einer wäßrigen Lösung aus 20 g Nitrendipin und 1,5 g Hydroxypropylcellulose Typ L granuliert. Nach Trocknen und Sieben wird das Granulat mit 1,5 g Magnesiumstearat vermisch und zu 310 mg schweren Tabletten mit einem Durchmesser von 9 mm verpreßt. Diese Tabletten werden dann mit einer wäßrigen Suspension aus Hydroxypropylmethylcellulose, Polyethylenglykol und Titaniumdioxid lackiert.

Beispiel 20

A) Kern

4 g kristallines Nisoldipin mit einem mittleren Teilchendurchmesser von 5 µm werden mit 8 g Lactose, 15 g quervernetztem PVPP und 12,3 g mikrokristalliner Cellulose gemischt und diese Mischung mit einer wäßrigen Lösung aus 1,8 g PVP und 0,8 g Natriumlaurylsulfat granuliert. Das getrocknete und gesiebte Granulat wird mit 0,1 g Magesiumstearat gemischt und zu 42 mg schweren Tablettenkernen mit einem Durchmesser von 5 mm verpreßt.

B) Mantelgranulat

46,5 g Hydroxypropylcellulose Typ L und 100 g Hydroxypropylcellulose Typ M werden mit 103 g Lactose gemischt und diese Mischung mit einer wäßrigen Suspension aus 16 g Nisoldipin und 1,5 g Hydroxypropylcellulose Typ L granuliert. Nach Trocknen und Sieben wird das Granulat mit 1 g Magnesiumstearat gemischt und zu 310 mg schweren Tabletten mit einem Durchmesser von 9 mm verpreßt. Die Tabletten werden anschließend mit einer wäßrigen Suspension aus Hydroxypropylmethylcellulose, Polyethylenglykol, Titaniumdioxid und rotem Eisenoxid (Lichtschutz) lackiert.

Beispiel 21

A) Kern

Die Tablettenkerne werden wie in Beispiel 20 hergestellt.

B) Mantelgranulat

92,5 g Hydroxypropylcellulose Typ L und 54 g Hydroxypropylcellulose Typ M werden mit 103 g Lactose vermischt und diese Mischung analog zu Beispiel 20 granuliert und anschließend auf die Tablettenkerne zu Manteltabletten mit einem Gewicht von 310 mg und einem Durchmesser von 9 mm verpreßt, welche anschließend wie in Beispiel 20 mit einer Lackschicht versehen werden.

Beispiel 22

A) Kern

Die Herstellung der Kerne erfolgt gemäß Beispiel 20 A).

B) Mantelgranulat

175 g Hydroxypropylcellulose Typ M und 74,5 g Lactose werden gemischt und mit einer wäßrigen Suspension von 16 g Nisoldipin und 1,5 g Hydroxypropylcellulose Type L granuliert. Anschliessend wird das Granulat getrocknet, gesiebt und mit 1 g Magnesiumstearat vermischt und zu 310 mg schweren Tabletten mit einem Durchmesser von 9 mm verpreßt, welche anschließend wie in Beispiel 20 angegeben lackiert werden.

## Patentansprüche

**1.** Feste Arzneizubereitungen mit lang anhaltender Wirkung in Form einer Manteltablette, enthaltend einen schwer löslichen Dihydropyridin-Wirkstoff der allgemeinen Formel I,

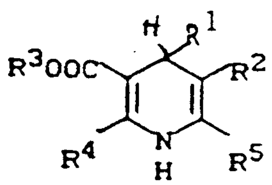

I,

in welcher

$R^1$ für einen Phenylrest steht, der durch ein oder zwei gleiche oder verschiedene Substituenten aus der Gruppe Nitro, Halogen oder Tifluormethyl substituiert ist, oder für einen Rest aus der Gruppe

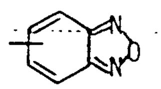

oder

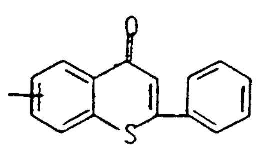

steht,

$R^2$ für eine Nitrogruppe steht oder für den Rest $COOR_6$ steht, wobei

$R_6$ Alkyl mit 1 bis 10 C-Atomen bedeutet, welches gegebenenfalls substituiert ist durch Alkoxy mit 1 bis 4 C-Atomen oder durch ein oder mehrere Halogene

oder wobei

$R^2$ gemeinsam mit $R^5$ für die Lactongruppe $-CO-O-CH_2-$steht,

$R^3$ für Alkyl mit 1 bis 10 C-Atomen steht, welches gegebenenfalls substituiert ist durch Alkoxy mit 1 bis 4 C-Atomen oder durch ein oder mehrere Fluor-Atome und

$R^4$ und $R^5$ gleich oder verschieden sind und jeweils für Alkyl mit 1 bis 4 C-Atomen, welches gegebenenfalls durch Hydroxy substituiert ist, stehen,

wobei die Manteltablette

a) aus einem Kern besteht, der 10 bis 40 % des Dihydropyridin-Wirkstoffs in schnell freisetzender Form enthält und

b) aus einem um den Kern liegenden Mantel besteht, der 60 bis 90 96 des Dihydropyridin-Wirkstoffs in langsam freisetzender Form enthält.

**2.** Arzneizubereitung gemäß Anspruch 1, dadurch gekennzeichnet, daß der Kern den Wirkstoff in amorpher Form oder in kristalliner Form mit einer maximalen mittleren Korngröße von 25 Um enthält, wobei bei Anwesenheit von kristallinem Wirkstoff der Kern zusätzlich leicht wasserlösliche Hilfsstoffe. Sprengmittel und/oder Netzmittel enthält.

**3.** Arzneimittelzubereitung gemäß Anspruch 1, dadurch gekennzeichnet, daß der langsam freisetzende Mantel 10 - 99% des Gesamtmantelgewichtes an hydrophilen gelbildenden Polymeren enthält.

**4.** Arzneimittelzubereitung gemäß Anspruch 1, dadurch gekennzeichnet, daß der Mantel als hydrophile gelbildende Polymere Methylcellulose, Hydroxypropylmethylcellulose, Hydroxypropylcellulose und/oder Natriumcarboxymethylcellulose enthält.

**5.** Arzneimittelzubereitung gemäß Anspruch 1, dadurch gekennzeichnet, daß sie als hydrophiles gelbildendes Polymer Hydroxypropylcellulose enthält.

**6.** Arzneimittelzubereitung gemäß Anspruch 1, enthaltend mindestens einen Wirkstoff aus der Gruppe Nifedipin, Nitrendipin, Nimodipin, Nisoldipin und Felodipin.

**7.** Verfahren zur Herstellung von festen Arzneizubereitungen mit lang anhaltender Wirkung in Form einer Manteltablette enthaltend einen schwer löslichen Dihydropyridin-Wirkstoff der allgemeinen Formel

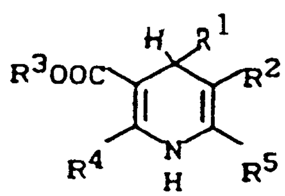

I,

in welcher

$R^1$ für einen Phenylrest steht, der durch ein oder zwei gleiche oder verschiedene Substituenten aus der Gruppe Nitro, Halogen oder Trifluormethyl substituiert ist, oder für einen Rest aus der Gruppe

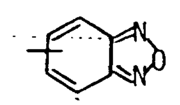

oder

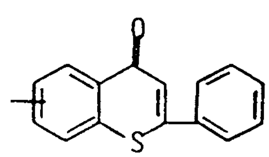

steht,

$R^2$ für eine Nitrogruppe steht oder für den Rest $COOR_6$ steht, wobei

$R_6$ Alkyl mit 1 bis 10 C-Atomen bedeutet, welches gegebenenfalls substituiert ist durch Alkoxy mit 1 bis 4 C-Atomen oder durch ein oder mehrere Halogene

oder wobei

$R^2$ gemeinsam mit $R^5$ für die Lactongruppe $-CO-O-CH_2$-steht,

$R^3$ für Alkyl mit 1 bis 10 C-Atomen steht, welches gegebenenfalls substituiert ist durch Alkoxy mit 1 bis 4 C-Atomen oder durch ein oder mehrere Fluor-Atome und

$R^4$ und $R^5$ gleich oder verschieden sind und jeweils für Alkyl mit 1 bis 4 C-Atomen, welches gegebenenfalls durch Hydroxy substituiert ist, stehen,

wobei die Manteltablette

a) aus einem Kern besteht, der 10 bis 40 % des Dihydropyridin-Wirkstoffs in schnell freisetzender

Form enthält und

b) aus einem um den Kern liegenden Mantel besteht, der 60 bis 90 % des Dihydropyridin-Wirkstoffs in langsam freisetzender Form enthält,

dadurch gekennzeichnet, daß man den schnell freisetzenden Kern herstellt, indem man den Wirkstoff mit anderen Hilfs- und Zuschlagstoffen in ein schnell freisetzendes Granulat überführt nach üblichen Granulationstechniken und anschliessend zu einem Kern preßt, der gegebenenfalls mit einer magensaftresistenten Schicht lackiert wird und dann auf diesen Kern ein Mantelgranulat, welches den Wirkstoff in langsam freisetzender Form enthält, auf einer Manteltablettenpresse verpreßt, wobei man das Mantelgranulat vorzugsweise durch Aufsprühen einer wäßrigen Suspension, welche den Wirkstoff mit einem Bindemittel enthält, auf feste Hilfsstoffe im Wirbelschichtgranulator herstellt, und wobei die erhaltenen Mantelkerntabletten gegebenenfalls noch mit einer Lackschicht umgeben werden, die ihrererseits bis zu maximal 20 Gewichtsprozent der gesamten Wirkstoffmenge der Zubereitung enthalten kann.

## Claims

**1.** Solid medicament preparations which have a long-lasting action in the form of a coated tablet and which contain a sparingly soluble dihydropyridine active compound of the general formula I

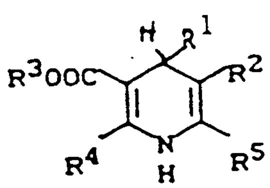

I

in which

$R^1$ represents a phenyl radical which is substituted by one or two identical or different substituents from the group comprising nitro, halogen and trifluoromethyl, or represents a radical from the group comprising

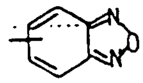

and

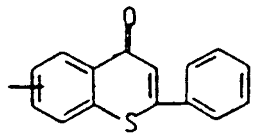

$R^2$ represents a nitro group or the radical $COOR_6$, in which

$R_6$ denotes alkyl having 1 to 10 C atoms which is optionally substituted by alkoxy having 1 to 4 C atoms or by one or more halogens,

or in which

$R^2$, together with $R^5$, represents the lactone group $-CO-O-CH_2$,

$R^3$ represents alkyl having 1 to 10 C atoms, which is optionally substituted by alkoxy having 1 to 4 C atoms or by one or more fluorine atoms and

$R^4$ and $R^5$ are identical or different and in each case represent alkyl having 1 to 4 C atoms, which is optionally substituted by hydroxyl,

where the coated tablet

a) consists of a core which contains 10 to 40% of the dihydropyridine active compound in rapid-release form and

b) consists of a coating around the core, this coating containing 60 to 90% of the dihydropyridine

active compound in slow-release form.

**2.** Medicament preparation according to Claim 1, characterized in that the core contains the active compound in amorphous form or in crystalline form having a maximum mean particle size of 25 µm, where the core additionally contains readily water-soluble auxiliaries, disintegrants and/or wetting agents in the presence of crystalline active compound.

**3.** Medicament preparation according to Claim 1, characterized in that the slow-release coating contains 10 - 99% of the total coating weight of hydrophilic gel-forming polymers.

**4.** Medicament preparation according to Claim 1, characterized in that the coating contains methylcellulose, hydroxypropylmethylcellulose, hydroxypropylcellulose and/or sodiumcarboxymethylcellulose as the hydrophilic gel-forming polymers.

**5.** Medicament preparation according to Claim 1, characterized in that it contains hydroxypropyl-cellulose as the hydrophilic gel-forming polymer.

**6.** Medicament preparation according to Claim 1, containing at least one active compound from the group comprising nifedipine, nitrendipine, nimodipine, nisoldipine and felodipine.

**7.** Process for the preparation of solid medicament preparations which have a long-lasting action in the form of a coated tablet and which contain a sparingly soluble dihydropyridine active compound of the general formula

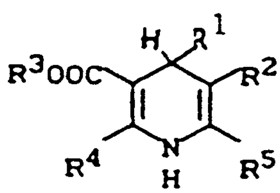

I,

in which

$R^1$ represents a phenyl radical which is substituted by one or two identical or different substituents from the group comprising nitro, halogen and trifluoromethyl, or represents a radical from the group comprising

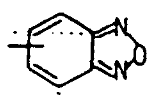

and

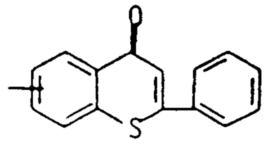

$R^2$ represents a nitro group or the radical $COOR_6$, in which

$R_6$ denotes alkyl having 1 to 10 C atoms which is optionally substituted by alkoxy having 1 to 4 C atoms or by one or more halogens,

or in which

$R^2$, together with $R^5$, represents the lactone group $-CO-O-CH_2$,

$R^3$ represents alkyl having 1 to 10 C atoms, which is optionally substituted by alkoxy having 1 to 4 C atoms or by one or more fluorine atoms and

$R^4$ and $R^5$ are identical or different and in each case represent alkyl having 1 to 4 C atoms,

which is optionally substituted by hydroxyl,

where the coated tablet

a) consists of a core which contains 10 to 40% of the dihydropyridine active compound in rapidrelease form and

b) consists of a coating around the core, this coating containing 60 to 90% of the dihydropyridine active compound in slow-release form,

characterized in that the rapid-release care is prepared by converting the active compound with other auxiliaries and additives into rapid-release granules by customary granulation techniques and subsequently pressing these granules to form a core which if appropriate is coated with a gastric juice-resistant layer, and then, in a coated-tablet press, pressing onto this core coating granules which contain the active compound in slow-release form, the coating granules preferably being prepared by spraying an aqueous suspension which contains the active compound and a binding agent onto solid auxiliaries in a fluidized-bed granulator, and the coated-core tablets obtained if appropriate being further surrounded with a coating layer which for its part can contain up to a maximum of 20 per cent by weight of the total quantity of active compound in the preparation.

**Revendications**

**1.** Préparations médicamenteuses solides à action persistant longtemps, sous forme d'un comprimé enrobé, contenant une substance active de dihydropyridine difficilement soluble et répondant à la formule générale I,

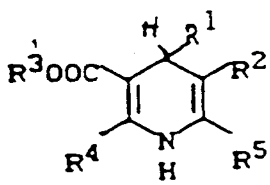

I,

dans laquelle

$R^1$ représente un radical phényle qui est substitué par un ou deux substituants identiques ou différents choisis parmi le groupe comprenant un groupe nitro, un atome d'halogène ou un groupe trifluorométhyle, ou bien un radical du groupe

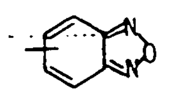

ou

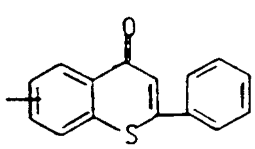

$R^2$ représente un groupe nitro ou le radical $COOR_6$, où

$R_6$ représente un groupe alkyle contenant de l à 10 atomes de carbone, qui est éventuellement substitué par un groupe alcoxy contenant de 1 à 4 atomes de carbone ou par un ou plusieurs atomes d'halogène

ou dans laquelle

$R^2$ représente, conjointement avec $R^5$, le groupe lactone $-CO-O-CH_2-$,

$R^3$ représente un groupe alkyle contenant de 1 à 10 atomes de carbone, éventuellement substitué par un groupe alcoxy contenant de 1 à 4 atomes de carbone ou par un ou

plusieurs atomes de fluor et

$R^4$ et $R^5$ sont identiques ou différents et représentent chacun un groupe alkyle contenant de 1 à 4 atomes de carbone, qui est éventuellement substitué par un groupe hydroxyle,

dans lesquelles le comprimé enrobé est constitué par :

a) un noyau qui contient de 10 à 40% de la substance active de dihydropyridine sous forme à libération rapide et

b) une enveloppe entourant le noyau, qui contient de 60 à 90% de la substance active de dihydropyridine sous forme à libération lente.

**2.** Préparation médicamenteuse selon la revendication 1, **caractérisée en ce que** le noyau contient la substance active sous forme amorphe ou sous forme cristalline avec une granulométrie moyenne maximale de 25 µm, dans laquelle, en présence d'une substance active cristalline, le noyau contient, en outre, des substances auxiliaires aisément solubles dans l'eau, des agents de dispersion et/ou des agents mouillants.

**3.** Préparation de médicament selon la revendication 1, **caractérisée en ce que** l'enveloppe à libération lente contient des polymères hydrophiles formateurs de gel à raison de 10 à 99% du poids total de l'enveloppe.

**4.** Préparation de médicament selon la revendication 1, **caractérisée en ce que** l'enveloppe contient, comme polymères hydrophiles formateurs de gel, de la méthylcellulose, de l'hydroxypropylméthylcellulose, de l'hydroxypropylcellulose et/ou de la carboxyméthylcellulose de sodium.

**5.** Préparation de médicament selon la revendication 1, **caractérisée en ce qu'**elle contient, comme polymère hydrophile formateur de gel, de l'hydroxypropylcellulose.

**6.** Préparation de médicament selon la revendication 1, contenant au moins une substance active choisie parmi le groupe comprenant la nifédipine, la nitrendipine, la nimodipine, la nisoldipine et la félodipine.

**7.** Procédé pour l'obtention de préparations médicamenteuses solides à action persistant longtemps, sous forme d'un comprimé enrobé contenant une substance active de dihydropyridine difficilement soluble et répondant à la formule générale,

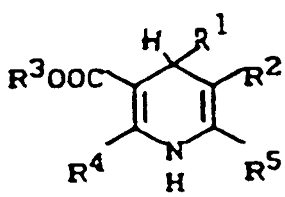

I,

dans laquelle

$R^1$ représente un radical phényle qui est substitué par un ou deux substituants identiques ou différents choisis parmi le groupe comprenant un groupe nitro, un atome d'halogène ou un groupe trifluorométhyle, ou bien un radical du groupe

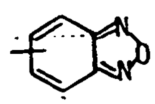

ou

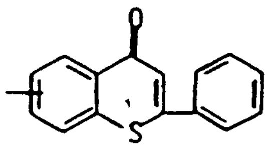

$R^2$ représente un groupe nitro ou le radical $COOR_6$, où

$R_6$ représente un groupe alkyle contenant de 1 à 10 atomes de carbone, qui est éventuellement substitué par un groupe alcoxy contenant de 1 à 4 atomes de carbone ou par un ou plusieurs atomes d'halogène

ou dans laquelle

$R^2$ représente, conjointement avec $R^5$, le groupe lactone $-CO-O-CH_2-$,

$R^3$ représente un groupe alkyle contenant de 1 à 10 atomes de carbone, éventuellement substitué par un groupe alcoxy contenant de 1 à 4 atomes de carbone ou par un ou plusieurs atomes de fluor et

$R^4$ et $R^5$ sont identiques ou différents et représentent chacun un groupe alkyle contenant de 1 à 4 atomes de carbone, qui est éventuellement substitué par un groupe hydroxyle,

dans lesquelles le comprimé enrobé est constitué par :

a) un noyau qui contient de 10 à 40% de la substance active de dihydropyridine sous forme à libération rapide et

b) une enveloppe entourant le noyau, qui contient de 60 à 90% de la substance active de dihydropyridine sous forme à libération lente.

**caractérisé en ce qu'**on prépare le noyau à libération rapide en transformant la substance active avec d'autres substances auxiliaires et d'addition en un granulé à libération rapide conformément à des techniques habituelles de granulation et ensuite, on le presse pour obtenir un noyau que l'on enrobe éventuellement d'une couche résistant au suc gastrique et on comprime alors sur ce noyau, un granulé d'enrobage qui contient la substance active sous forme à libération lente, sur une presse pour comprimés enrobés, dans laquelle on prépare le granulé d'enrobage de préférence par aspersion d'une suspension aqueuse qui contient la substance active avec un agent liant, sur des substances auxiliaires solides dans un granulateur en lit fluidisé et dans laquelle on enrobe éventuellement encore les comprimés à noyau enrobé obtenus, avec une couche de laque qui, à son tour, peut contenir jusqu'à 20% en poids au maximum de la quantité totale de substance active de la préparation.

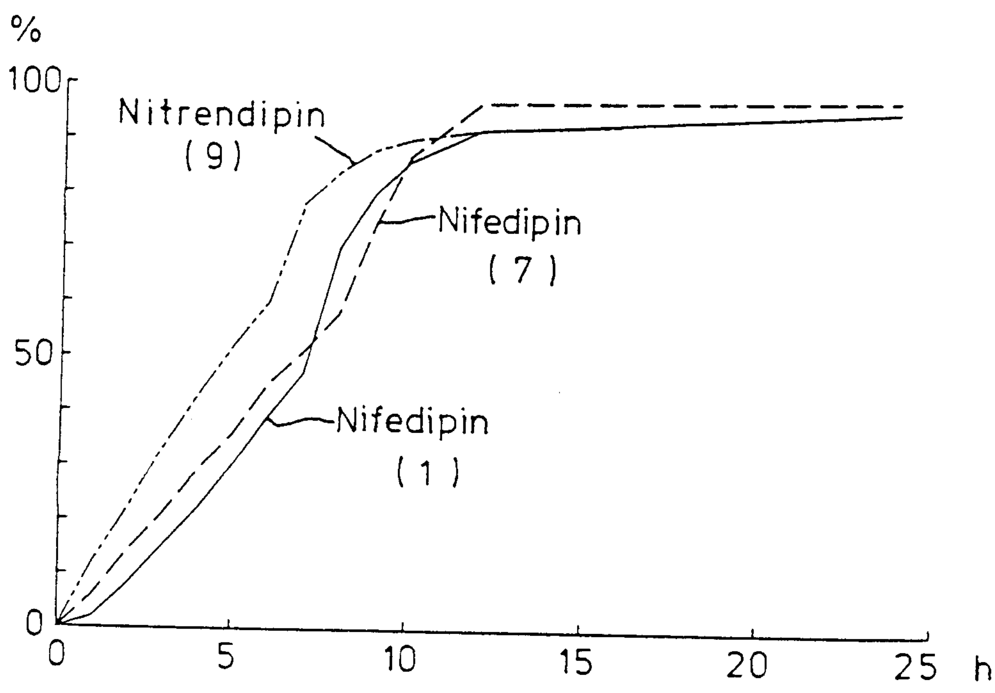


FIG. 1